Europäisches Patentamt

European Patent Office

Office européen des brevets

(11) Publication number: **0 146 368**
**A2**

# (12) EUROPEAN PATENT APPLICATION

(21) Application number: **84308757.8**

(22) Date of filing: **14.12.84**

(51) Int. Cl.⁴: **C 12 N 15/00**

(30) Priority: **16.12.83 GB 8333659**

(43) Date of publication of application: **26.06.85**
**Bulletin 85/26**

(84) Designated Contracting States: **AT BE CH DE FR GB IT
LI LU NL SE**

(71) Applicant: **Pfizer Limited, Ramsgate Road, Sandwich
Kent CT13 9NJ (GB)**

(84) Designated Contracting States: **GB**

(71) Applicant: **Pfizer Corporation, Calle 15 1/2 Avenida
Santa Isabel, Colon (PA)**

(84) Designated Contracting States: **BE CH DE FR IT LI LU
NL SE AT**

(72) Inventor: **Hunter, Iain Startk, Dr., 6 Lammermuir
Gardens, Baljaffrey Bearsden Glasgow G61 (GB)**

(74) Representative: **Graham, Philip Colin Christison et al,
Pfizer Limited Ramsgate Road, Sandwich, Kent
CT13 9NJ (GB)**

(54) Cosmid cloning vectors.

(57) A bi-functional, hybrid cosmid cloning vector capable of
replication in both Eschericia coli and in Streptomycetes is con-
structed by (a) cleaving a broad host-range Streptomycete
plasmid vector at a particular endonuclease-sensitive restriction site; (b) ligating DNA from a plasmid from an E. coli host
to the cleaved plasmid to form a hybrid vector having two of
said sites; and (c) inserting cohesive-end sequences excised
from a suitable source into said hybrid vector by cleaving the
latter at a suitable site and ligating the cohesive-end sequences
to the cleaved hybrid vector. The particular cosmid cloning
vector pPZ74 is described. Such vectors are useful for the con-
struction of gene libraries in E. coli, which can then be trans-
ferred back into Streptomyces without further DNA manipula-
tion.

This invention relates to cosmid cloning vectors and in particular to a cosmid vector capable of gene cloning in a wide variety of Streptomycetes, including the oxytetracycline-producing organism Streptomyces rimosus.

Cosmids are plasmids having within their structure cohesive-end sites, which enable them to be used as vectors for gene cloning in conjunction the phage packaging technique, described by Collins and Hohn in Proc. Nat. Acad. Sci. 75, 4242-4246 (1978) and also in British Patent no. 2017754 and U.S. Patent 4304863.

The present invention provides a novel hybrid cosmid cloning vector, which has been constructed in such a way as to be bi-functional, i.e. it is capable of replication in both Escherichia coli and in Streptomycetes. Gene cloning can therefore be carried out in E. coli using this vector and the packaging technique of Collins and Hohn, and the vector can then be transformed into Streptomycetes as well as into E. coli.

The advantage of gene-cloning with a cosmid vector using the packaging technique of Collins and Hohn is that relatively large DNA inserts can be used. In the construction of genomic DNA libraries, the number of clones to be screened is related both directly to genome size and inversely to the average size of the DNA inserts. It follows therefore that to minimise the number of clones to be screened, the size of the DNA insert should be maximised. Streptomyces have a genome complexity of $10^4$ kb. The binomial theorem predicts that a genomic library prepared with Streptomycete DNA of average size 5kb - typical for cloning experiments - would need to contain 6000 independent clones to have a 95% probability that all of the genomic sequences were represented. Increasing the size of the DNA insert to 30kb would reduce the number of clones to be screened to 1000. This reduction would be particularly useful when no selection strategy (e.g. phenotypic expression in a blocked mutant or auxotroph) was available to screen for the clone of interest and where labour-intensive screens using enzyme or product assays have to be employed. However, it is difficult technically to prepare libraries with such large inserts using conventional vectors. The bi-functional cosmid vector of this invention will select for clones with insert size 25-40kb and by using it gene libraries can be constructed in E. coli and transferred back into Streptomyces without further DNA manipulation.

More specifically, the invention provides a novel hybrid cosmid designated pPZ74.

The cosmid pPZ74 is constructed according to the invention from a plasmid, pPZ12, obtainable from a strain of Streptomyces albus AT95 containing it, which was deposited for the purposes of patent procedure under the Budapest Treaty in the Culture Collection of the National Collection of Industrial and Marine Bacteria at the Torry Research Station, in Aberdeen, Scotland, on 12th December, 1983 and has received the accession number NCIB 11936.

In general, to construct a hybrid cosmid cloning vector according to the invention, a broad host-range Streptomycete plasmid vector (e.g. pPZ12) is cleaved at a particular endonuclease-sensitive, restriction site (e.g. a Pst I - sensitive site) and DNA from a plasmid from an E. coli host (e.g. pBR325) is ligated to the cleaved plasmid to form a hybrid, bi-functional vector having two of such sites. Cohesive-end sequences excised from a suitable source (e.g. plasmid pBTI-1) are then inserted into the hybrid vector by cleaving the latter at a suitable site (e.g. one of the Pst I - sensitive sites) and ligating the cohesive-end sequences to the cleaved hybrid vector, thus forming the hybrid cosmid of the invention.

Preferably, the Streptomycete plasmid vector contains a single restriction site which will be unique in the final hybrid cosmid of the invention and which will provide a suitable cleavage site for insertion of donor DNA having the desired 25-40kb size. Such DNA will have been cut (restricted) by a restriction enzyme (such as Sau 3A or Taq I) which cuts that DNA frequently thereby generating, by partial digestion, random DNA fragments of donor organism, including fragments of 25-40kb. The restriction enzyme chosen to digest the donor DNA will of course be such as to provide fragments which can be inserted at the unique restriction site in the hybrid cosmid vector. For example, the restriction enzyme Sau 3A will provide DNA fragments which can be inserted at a unique Bgl II - sensitive site in the cosmid vector. Thus, a Bgl II site may be inserted in place of the unique Sst I site in the Streptomycete plasmid pPZ12, before insertion of pBR325 DNA, to provide the desired unique Bgl II site in the hybrid cosmid of the invention.

The techniques used for cleaving plasmid DNA at specific sites and ligating DNA from another source to the cleaved DNA, and the designations of the particular enzymes used, are well known to those skilled in the art and are described, for example, in the laboratory manual "Molecular Cloning" by T. Maniatis et al (Cold Spring Harbor Laboratory, 1982).

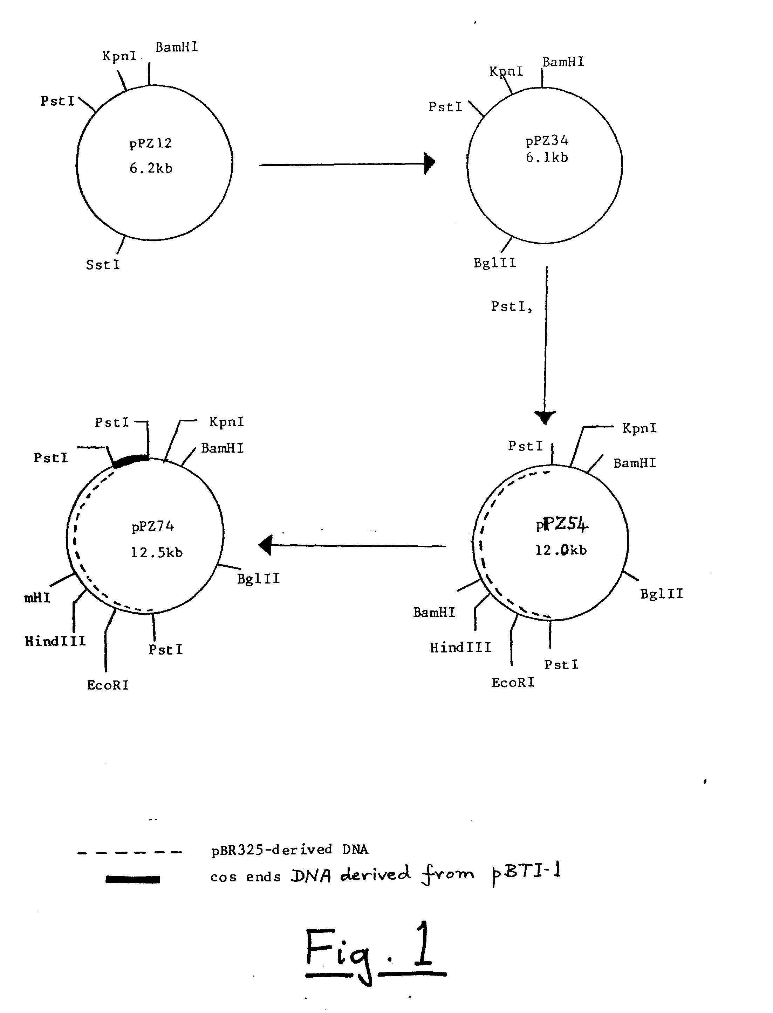

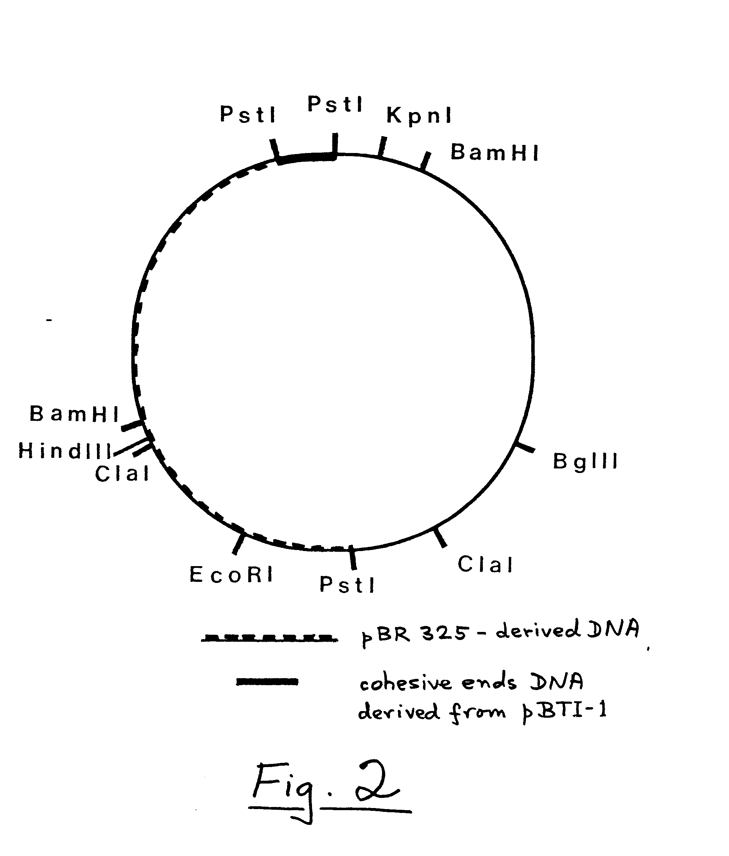

The following is an Example of the construction of a hybrid cosmid cloning vector according to the invention, namely pPZ74, which is also illustrated in Fig. 1 of the accompanying drawings. Temperatures are given °Celsius.

- 5 -

EXAMPLE

(a)  Preparation of plasmid pPZ34

Plasmid pPZ12 is isolated from Streptomyces albus AT95 using the method described by Chater et al, Current Topics in Microbiology and Immunology, 1982, 96, 69-95. The isolated plasmid DNA is finally dissolved in water to produce a concentration of 1μg of pPZ12 per 5μl of water. Its unique Sst I site is replaced by a Bgl II site by the following technique. 10μg of the plasmid pPZ12 in 50μl water are incubated with 50 units of the restriction enzyme Sst I (Bethesda Research Laboratories Inc. (BRL), catalogue no. 5222SA) in the assay buffer specified by the supplier, at 37° for 2h, in a total reaction volume of 250μl. The DNA is precipitated by adding 2½ volumes (625μl) of absolute ethanol, centrifuged at 10,000g for 10 min and dried under vacuum. The DNA is then dissolved in 20μl water and incubated with 0.5 units of nuclease Bal-31 (New England Biolabs Inc., Catalogue no. 213) in the assay buffer specified by the supplier at 30° for 3 min in a total reaction volume of 100μl. The reaction is terminated by adding 10μl of a 0.2M solution (pH 8) of EGTA (ethylene glycol bis [β-aminoethyl ether]-N,N$^1$- tetra acetic acid) and the DNA precipitated with ethanol, centrifuged and dried, as before. The DNA is again dissolved in 20μl water and incubated with 5 units of DNA - polymerase (Klenow, "large fragment" : Boehringer Mannheim GmbH, Catalogue no. 152 692) in the buffer specified by the supplier in a total volume of 100μl at 20° for 30 min. The reaction is terminated by heating at 65° for 10 min and the DNA precipitated with ethanol, centrifuged and dried, as before. The DNA is redissolved in 20μl water and

ligated with a 50-fold molar excess of Bgl II linker (New England Biolabs, Cat. No. 1001) in accordance with the supplier's instructions. The reaction mixture is then used to transform protoplasts of <u>Streptomyces albus</u>, by the method described in Example I (c) of our patent application (Ref. PLC 387 filed concurrently herewith), the contents of which are incorporated herein by reference, but using <u>Streptomyces albus</u> instead of mutagenised <u>S. rimosus</u> 4018S. The host cells are regenerated and thiostrepton-resistant colonies are selected and purified as described in Example I (d) and (e) of that application. These colonies should contain the plasmid pPZ34, which includes a unique Bgl II - sensitive site; this is confirmed by isolating plasmid DNA from the colonies (by the method already described for pPZ12) and showing that it is cleaved by the restriction enzyme Bgl II.

(b)  <u>Preparation of plasmid pPZ54</u>

10μg of plasmid pPZ34 dissolved in 50μl water are incubated with 50 units of the restriction enzyme Pst I (BRL Cat. No. 5215SA) in the assay buffer specified by the supplier, at 37° for 2h, in a total reaction volume of 250μl. The cleaved pPZ34 plasmid DNA is precipitated with ethanol, centrifuged and dried, as before. 10μg of the plasmid pBR325 (prepared as described on pp 92-94 of the laboratory manual "Molecular Cloning", already referred to) are also digested with the restriction enzyme Pst I, as described above, precipitated with ethanol, centrifuged and dried, as before. This DNA is redissolved in 100μl of 0.1M glycine buffer (pH 10.2) and incubated with 5 units of calf intestinal alkaline phosphatase (Boehringer Mannheim Cat. No. 108 154) for 30 min at 37°. The reaction is terminated by heating at 65° for 10 min and precipitated with ethanol, centrifuged and dried, as before. The DNA is redissolved in 20μl water and mixed with the cleaved pPZ34 plasmid DNA, also redissolved in 20μl water; and incubated with T4 DNA ligase (BRL, Cat. No. 5224SB) in the assay buffer specified by the supplier, at a total DNA concentration of 20μg per ml, at 14° for 16h. The DNA is precipitated with ethanol, centrifuged and dried, as before, redissolved in 20μl water and used to transform competent cells of E. coli strain MC1061 (Casadaban et al, J. Mol. Biol. 1980, 138, 179) by the method described on pp.250-1 of the laboratory manual "Molecular Cloning", already referred to. Chloramphenicol-resistant, tetracycline-resistant, ampicillin-sensitive colonies are selected which should contain the plasmid pPZ54, which now includes plasmid DNA from pBR325 as well as from pPZ34; this is confirmed by isolating plasmid DNA from the colonies (as already described for pPZ12) and showing that the DNA is susceptible to cleavage by restriction enzymes in the predicted manner.

(c) Preparation of hybrid cosmid pPZ74

10μg of plasmid pPZ54 dissolved in 50μl water are incubated with 2 units of the restriction enzyme Pst I in assay buffer (as previously specified) at 37° for 30 min in a total reaction volume of 250μl. The reaction is terminated by heating at 65° for 10 min and the reaction mixture loaded onto an 0.8% agarose gel and subjected to electrophoresis as described on pp 150-167 of the laboratory manual "Molecular Cloning" already referred to. The 12kb linear DNA component of the mixture is recovered from the gel, redissolved in 0.1M glycine buffer, incubated with calf intestinal alkaline phosphatase, the reaction terminated and the DNA recovered and redissolved in 20μl water, all as previously

described in (b). 20µg of plasmid pBTI-1 (Boehringer Mannheim, Cat. No. 674 222) is digested with the restriction enzyme Pst I in assay buffer (as previously specified), loaded onto a 8.0% polyacrylamide gel and subjected to electrophoresis, as described on pp. 173-178 of the laboratory manual, "Molecular Cloning", already referred to. The smaller fragment is recovered from the gel, precipitated with ethanol, centrifuged and dried, as before, and redissolved in 20µl water. This is then mixed with the solution of alkaline phosphatase-treated 12kb linear DNA component and incubated with T4 DNA ligase, as previously described in (b), and the DNA precipitated with ethanol, centrifuged and dried, as before. This is then used to transform E. coli MC1061, as described in (b) and chloramphanicol-resistant, tetracycline-resistant, ampicillin-sensitive colonies are selected which should now contain the hybrid cosmid pPZ74 according to the invention. This is confirmed by isolating plasmid DNA from the colonies (as already described for pPZ12) and showing that the DNA is susceptible to cleavage by restriction enzymes in the predicted manner.

A restriction map of the hybrid cosmid pPZ74 is shown in Fig. 2 of the accompanying drawings.

CLAIMS

1.  A bi-functional, hybrid cosmid cloning vector capable of replication in both Escherichia coli and in Streptomycetes.

2.  The cosmid cloning vector identified herein as pPZ74.

3.  A method of constructing a cosmid cloning vector capable of replication in both Escherichia coli and in Streptomycetes, which comprises the steps of:

(a)  cleaving a broad host-range Streptomycete plasmid vector at a particular endonuclease-sensitive restriction site;

(b)  ligating DNA from a plasmid from an E. coli host to the cleaved plasmid to form a hybrid vector having two of said sites; and

(c)  inserting cohesive-end sequences excised from a suitable source into said hybrid vector by cleaving the latter at a suitable site and ligating the cohesive-end sequences to the cleaved hybrid vector.

4.  A method according to claim 3, in which said Streptomycete plasmid vector contains a single restriction site which will be unique in said cosmid cloning vector.

5.  A method according to claim 4, in which said single restriction site is a Bgl II site.

6.  A method according to claim 5, in which said vector is the plasmid pPZ12 into which a Bgl II site has been inserted in place of the single SstI site therein.

7.  A method according to any of claims 3 to 6, in which said particular endonuclease-sensitive restriction site at which said Streptomycete plasmid vector is cleaved in step (a) is a PstI-sensitive site.

- 10 -

8.    A method according to any of claims 3 to 7, in which said DNA from a plasmid from an E. coli host used in step (b) is DNA from the plasmid pBR325.

9.    A method according to any of claims 3 to 8, in which said cohesive end sequences used in step (c) are excised from the plasmid pBTI-1.

10.   A cosmid cloning vector capable of replication in both Escherichia coli and in Streptomycetes, constructed by a method as claimed in any of claims 3 to 9.

PFIZER LTD.(PLC 386)

0146368

- - - - - - -  pBR325-derived DNA

━━━━  cos ends DNA derived from pBTI-1

Fig. 1

Pstl    Pstl    Kpnl

BamHI

BamHI

HindIII

Clal

BglII

Clal

EcoRI    Pstl

------- pBR 325 - derived DNA

——— cohesive ends DNA
derived from pBTI-1

Fig. 2